# EUROPEAN PATENT SPECIFICATION

(11) **EP 3 438 236 B1**
(45) Date of publication and mention of the grant of the patent: **24.08.2022**
(21) Application number: 17773807.7
(22) Date of filing: 17.02.2017
(51) Int. Cl.: C12M 1/26, C12M 1/00, C12N 1/00, G01N 33/00, C12N 5/00

(54) **CELL CULTURE CONTAINER, CELL CULTURE SYSTEM, CELL CULTURE KIT AND CELL CULTURE METHOD**
ZELLKULTURBEHÄLTER, ZELLKULTURSYSTEM, ZELLKULTURKIT UND ZELLKULTURVERFAHREN
RÉCIPIENT DE CULTURE DE CELLULES, SYSTÈME DE CULTURE DE CELLULES, TROUSSE DE CULTURE DE CELLULES ET PROCÉDÉ DE CULTURE DE CELLULES

(30) Priority: 28.03.2016 JP 2016064701
(43) Date of publication of application: 06.02.2019
(73) Proprietor: Sony Group Corporation, Tokyo 108-0075 (JP)
(72) Inventor: MATSUMOTO Masahiro, Tokyo 108-0075 (JP)
(74) Representative: D Young & Co LLP
(86) International application number: PCT/JP2017/005844
(87) International publication number: WO 2017/169259

(56) References cited:
- EP-A1- 2 500 424
- WO-A1-92/11355
- WO-A1-03/074691
- WO-A1-2011/058721
- WO-A1-2012/141202
- WO-A1-2013/002311
- JP-A- 2000 125 848
- JP-A- 2004 089 136
- JP-A- 2004 194 720
- JP-A- 2010 008 256
- JP-A- 2010 252 635
- JP-A- 2011 239 734
- JP-A- 2015 116 152
- SHINYA ARIYASU ET AL: "Selective Capture and Collection of Live Target Cells Using a Photoreactive Silicon Wafer Device Modified with Antibodies via a Photocleavable Linker", LANGMUIR, vol. 28, no. 36, 29 August 2012 (2012-08-29), pages 13118-13126, XP055559454, US ISSN: 0743-7463, DOI: 10.1021/la302393p
- ZHU H ET AL: "Catch and release cell sorting: Electrochemical desorption of T-cells from antibody-modified microelectrodes", COLLOIDS AND SURFACES. B, BIOINTERFACES, ELSEVIER, AMSTERDAM, NL, vol. 64, no. 2, 15 July 2008 (2008-07-15), pages 260-268, XP022681262, ISSN: 0927-7765, DOI: 10.1016/J.COLSURFB.2008.02.010 [retrieved on 2008-03-10]
- YAMAGUCHI, S. et al.: "Photocontrollable Dynamic Micropatterning of Non-adherent Mammalian Cells Using a Photocleavable Poly(ethylene glycol) Lipid", Angew. Chem. Int. Ed., vol. 51, 2012, pages 128-131, XP055602106,
- KOETTING, M.C. et al.: "Stimulus-responsive hydrogels: Theory, modern advances, and applications", Mater. Sci. Eng. R, vol. 93, 2015, pages 1-49, XP055602110,
- ARIYASU, S. et al.: "Selective Capture and Collection of Live Target Cells Using a Photoreactive Silicon Wafer Device Modified with Antibodies via a Photocleavable Linker", Langmuir, vol. 28, 2012, pages 13118-13126, XP055559454, DOI: doi:10.1021/la302393p
- SHIN, D-S. et al.: "Photolabile micropatterned surfaces for cell capture and release", Chem. Commun., vol. 47, no. 43, 2011, pages 11942-11944, XP055602112,
- WEGNER, S.V. et al.: "Photoallergene linker for the patterning of bioactive molecules", Sci. Rep., vol. 5, no. 18309, 2015, pages 1-7, XP055602119,

## Description

### Technical Field

The present invention relates to a cell culture container, a cell culture system, a cell culture kit, and a cell culture method.

### Background Art

In the related art, it is needed to sort cells, culture specific cells, and easily recover the cells without damaging the cultured cells.

For example, Patent Literature 1 discloses a cell-culturing cuvette using, as an adhesive surface of an anchorage-dependent cell, a photo-responsive composition having a property of differential physical properties based on light irradiation. According to Patent Literature 1, cells are cultured by using the cell-culturing cuvette. If another cell type invades therein during culturing, light can be irradiated on the positions of the invading cells to release and remove the invading cells. Only the specific cell type will be left on a cell-adhesive surface, and continuously cultured. After the specific cell type is cultured, the cell-adhesive surface is irradiated with light and the cultured cells are recovered.

In addition, Patent Literature 2 discloses a method for analyzing and fractionating cells by film-forming a photo-controllable cell-adhesive material in which a cell-adhesive material is bonded to a cell-non-adhesive material through a photo-dissociable group. According to Patent Literature 2, a photodissociation reaction irreversibly changes a cell-adhesive substrate into a cell-non-adhesive substrate, showing excellence in the adhesion selectivity between the cells and the substrate, and the purity, recovery rate, and the like of cells can be enhanced.

In the meantime, according to the technology widely performed in the related art, target cells are gene transfected and then cultured. The apparatuses that can perform the cell processing in series have been already developed. For example, MACS Prodigy (registered trademark) Miltenyi Biotec Inc.) is commercially available.

The publication by Ariyasu S. et al., Langmuir, vol. 28, no. 36, 29 August 2012, pages 13118-13126 discloses a device for selectively capturing cells comprising a cell culture container which contains a silicon wafer at the bottom of the container. A photocleavable ANP-linker molecule (3-amino-3-(2-nitrophenyl)propionic acid) is bonded to the silicon wafer, wherein the ANP-linker's second end is attached to an anti-HEL-IgG antibody that binds HEL-presenting (hen egg lysozyme) SP2/O target cells. The target cells can be fluorescently labelled and can be detached from the culture dish by application of a light stimulus.

In Zhu H. et al., Colloids and Surfaces B: Biointerfaces, vol. 64, no. 2, 15 July 2008, pages 260-268, a device comprising a glass dish with a spotted pattern of a gold (Au) layer is disclosed. The Au-areas have the function of microelectrodes. Antibodies which recognise and bind T-cells are linked to the Au-microelectrodes via COOH-terminated thiols. After T-cells have bound selectively to the antibodies, the cells can be released by applying a reductive potential which disrupts the thiols. The integration of this set-up into a microfluidic device is suggested for the selective capture of specific cells, their release for further analysis and reculturation.

### Citation List

### Patent Literature

Patent Literature 1: Japanese Patent No. 3975266
Patent Literature 2: International Publication No. WO 2011/058721

### Disclosure of Invention

### Technical Problem

Patent Literature 1 and Patent Literature 2 propose the technology that cell-adhesive/cell-non-adhesive substrates can be optically controlled to sort, culture, recover cells, but do not propose the technology about cell processing.

In addition, if the technologies of Patent Literature 1, Patent Literature 2 and the like are used, once a cell density is increased during culturing, cultured cells have to be moved from a narrow culturing container to a wide culturing container for further culturing. There is a problem that moving the cultured cells takes times and efforts, and a stress and a damage caused by the movement are applied to the cells.

Further, the apparatus that transfects the genes into the target cells and cultures the target cells has a complex structure, and includes a cell sorter, culture part, and the like, each of which is connected via a tube. Thus, there is a problem that the cultured cells move through the tube, receive a stress and a damage, and are lost.

Still further, culturing the cells is preferably performed in a wide space. It is conceivable that activating the cells and transfecting the genes into the cells are preferably performed in a narrow space under the high concentration conditions.

In addition, a flow cytometer type cell sorter is often used. In the flow cytometer type sorter, beads are flowed for optical axis adjustment. However, for a clinical application, GMP grade beads should be prepared. Furthermore, there is a need to show that the beads are not included in a final product or, if included, do not affect on a human body.

Further, in the flow cytometer type sorter, it needs to flow a cell sample for gate adjustment in order to determine whether or not the cells are fractionated. There is also a problem that the cells are not recovered and discarded.

The present technology is made in view of the above-mentioned circumstances, and it is a main object of the present technology to provide a cell culture container and the like that can perform cell sorting, culturing, cell processing, and the like in one space and a volume of the space can be varied to suit respective steps.

### Solution to Problem

In order to solve the above-described problems, the present technology provides a cell culture container comprising: first molecules each bondable to target cells to be cultured, the first molecules being immobilized to the container via a stimulus degradable linker, the container having a volume being variable.

The stimulus degradable linker may be a photodegradable linker.

Also, the first molecules are immobilized to the container in an array having a spot with a size for bonding to one target cell to be cultured.

Also, each of the first molecules each may bond to a different kind of the target cells to be cultured for the spot.

Further, the cell culture container may have a gas permeability.

Further, the first molecules may be selected from the group consisting of an oleyl group, an antibody, an aptamer, and a molecular recognition polymer.

In addition, the cell culture container according to the present technology may have a connector that is connected to one selected from the group consisting of
a culture cell injection unit that injects solution containing the target cells,
a second molecule feeder that feeds labelled second molecules bondable to the target cells,
an activator feeder that feeds an activator of activating the target cells,
a gene feeder that feeds genes to be transfected to the target cells,
a culture solution feeder that feeds culture solution of the target cells,
a cleaning solution feeder that feeds cleaning solution of cleaning away an unnecessary material,
a waste solution reservoir that reserves the unnecessary material and the cleaning solution, and
a cultured cell recovery unit that recovers cultured cells of the target cells.

In addition, the present technology provides a cell culture system, including:
a cell culture container, including
first molecules each bondable to target cells to be cultured, the first molecules being immobilized to the container via a stimulus degradable linker, the container having a volume being variable; and a stimulus imparting device that imparts a stimulus to the stimulus degradable linker.

The stimulus imparting device may include a light source, an excitation filter, an emission filter, and an image sensor.

Also, the stimulus imparting device may include a stimulus controller that controls stimulation of the stimulus degradable linker for a spot of the first molecule.

Furthermore, the present invention provides a cell culture kit, including:
a cell culture container, including
first molecules each bondable to target cells to be cultured, the first molecules being immobilized to the container via a stimulus degradable linker, the container having a volume being variable and solution selected from the group consisting of sample solution containing the target cells to be cultured,
reagent solution containing labelled second molecules bondable to the target cells, activator solution containing an activator that activates the target cells, gene transfection solution containing genes transfected to the target cells, culture solution of the target cells, and cleaning solution that cleans away unnecessary material.

Furthermore, the present technology provides a cell culture method, including the steps of:
injecting a sample containing target cells to be cultured into a cell culture container, including first molecules each bondable to target cells, the first molecules being immobilized to the container via a stimulus degradable linker, the container having a volume being variable; applying labelled second molecules being bondable to the target cells to be cultured to the target cells to be cultured; sorting the target cells by the label and removing cells other than the target cells; and culturing the target cells to be cultured.

The cell culture method may further include the steps of:
activating the target cells to be cultured; and/or
transfecting genes to the target cells to be cultured.

In addition, at least one of the steps may include changing a volume of the cell culture container.

### Solution to Problem

### Advantageous Effects of Invention

According to the present technology, in a series of cell sorting, cell culturing, cell processing and the like, a stress, a damage, and the like applied on the cultured cells can be decreased.

It should be noted that the effects described here are not necessarily limitative and may be any of effects described in the present disclosure.

### Brief Description of Drawings

[Fig. 1] Fig. 1 is a schematic diagram of a cell culture system according to the present technology.
[Fig. 2] Fig. 2 is schematic diagram of a culture container according to the present technology.
[Fig. 3] Fig. 3 is a schematic diagram of first molecules immobilized according to the present technology.
[Fig. 4] Fig. 4 is a schematic diagram showing a structural example of the cell culture container according to the present technology.
[Fig. 5] Fig. 5 is a schematic diagram showing a structural example of a stimulus imparting device according to the present technology the present technology.
[Fig. 6] Fig. 6 is a block diagram showing steps of an embodiment according to the present technology.
[Fig. 7] Fig. 7 is schematic diagrams showing the cell culture container inside according to the embodiment the present technology.
[Fig. 8] Fig. 8 is schematic diagrams showing a volume variation of the cell culture container the present technology.
[Fig. 9] Fig. 9 is schematic diagrams showing movements of the cell culture container according to the present technology.
[Fig. 10] Fig. 10 is schematic diagrams showing movements of the cell culture container according to the present technology.

### Mode(s) for Carrying Out the Invention

Hereinafter, the present technology will be described further in detail with reference to Examples. The embodiments of the present technology will be described in the following order.
1. Cell culture system
   1-1. Cell culture container
   1-2. Stimulus imparting device
2. Embodiments
3. Cell culture kit

### <1. Cell culture system>

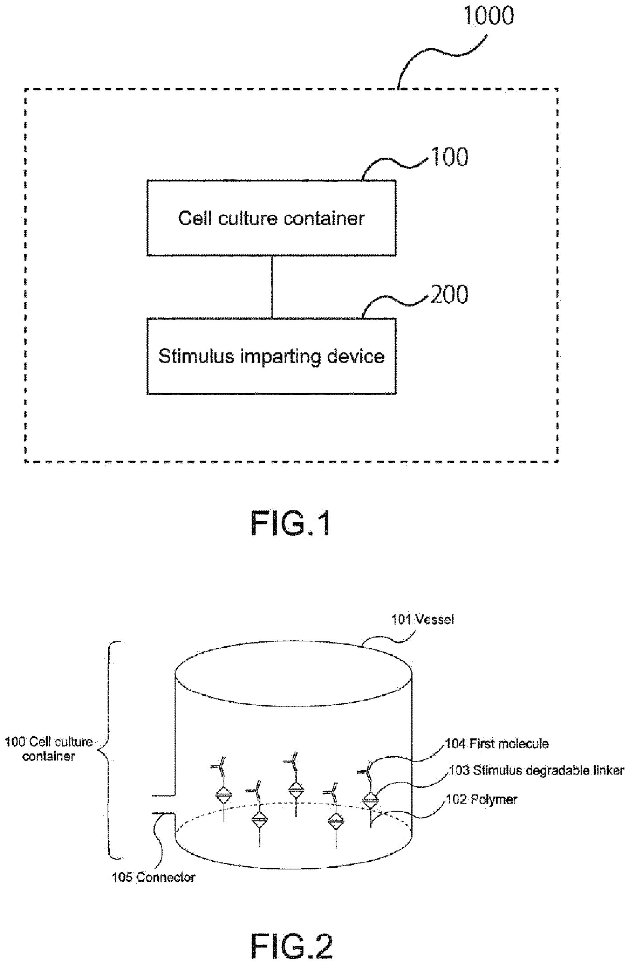

Fig. 1 shows a cell culture system according to the present technology.

A cell culture system 1000 includes a cell culture container 100 and a stimulus imparting device 200.

In the cell culture container 100, first molecules bondable to target cells to be cultured are immobilized to the container via stimulus degradable linkers. The cell culture container 100 has a variable volume that may be increased or decreased.

The stimulus imparting device 200 imparts corresponding stimuli to the stimulus degradable linkers. For example, in a case where the stimulus degradable linkers are photodegradable linkers, the stimulus imparting device 200 irradiates the stimulus degradable linkers with light.

### <1-1. Cell culture container>

Fig. 2 shows a schematic diagram of the cell culture container 100.

In a case where the target cells to be cultured are injected into the vessel 101, the cell culture container 100 traps the target cells to be cultured with the first molecules 104 immobilized thereto. The trapped target cells to be cultured are cultured in the vessel 101.

Inside the vessel 101 of the cell culture container 100, the first molecules 104 are immobilized via polymers 102 and stimulus degradable linkers 103, for example. The polymers 102 may be omitted and the stimulus degradable linkers 103 may be used for direct immobilization. The immobilization is not limited to a bottom surface of the vessel 101 and may be on an inner wall of the vessel 101. In a case where the vessel 101 has a planar or three-dimensional inner structure inside, the first molecules 104 may be immobilized on a surface of the structure. It is desirable that a material to make cells easy to live (for example, collagen, fibroblast, or the like) is coated on an inside surface of the vessel 101.

In a case where the polymers 102 are used, preferable polymers do not apply stress on the cells and have nontoxicity, biocompatibility, or the like. Examples include polyethylene glycol (PEG) and 2-methacryloyloxyethyl phosphorylcholine polymer (MPC polymer).

In a case where the polymers 102 are used, the stimulus degradable linkers 103 are bonded to respective ends opposite to ends bonded the vessel 101. The stimulus degradable linkers are connection molecules that are degraded by specific external stimuli. Examples include linkers degraded by light having a specific wavelength, linkers degraded by an enzyme, linkers degraded by temperature and the like.

The stimulus degradable linkers are not especially limited. From the viewpoints that it is possible to control for a single cell and that a degradation time is short, the photodegradable linkers are preferably used.

The photodegradable linkers are molecules having a structure that are degraded by a specific wavelength.

Examples includes: a methoxynitrobenzyl group, a nitrobenzyl group (Japanese Unexamined Patent Application Publication No.2010-260831), a parahydroxyphenacyl group (Tetrahydron Letters, 1962, vol. 1, p. 1), a 7-nitroindolin group (Journal of American Chemical Society, 1976, vol. 98, p. 843), a 2-(2-nitrophenyl)ethyl group (Tetrahydron, 1997, vol. 53, p. 4247) and (coumarin-4-yl)methyl group (Journal of American Chemical Society, 1984, vol. 106, p. 6860), and the like.

In addition, Table 1 shows structural formulae of the photodegradable linkers that can be used in the present technology.

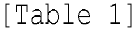

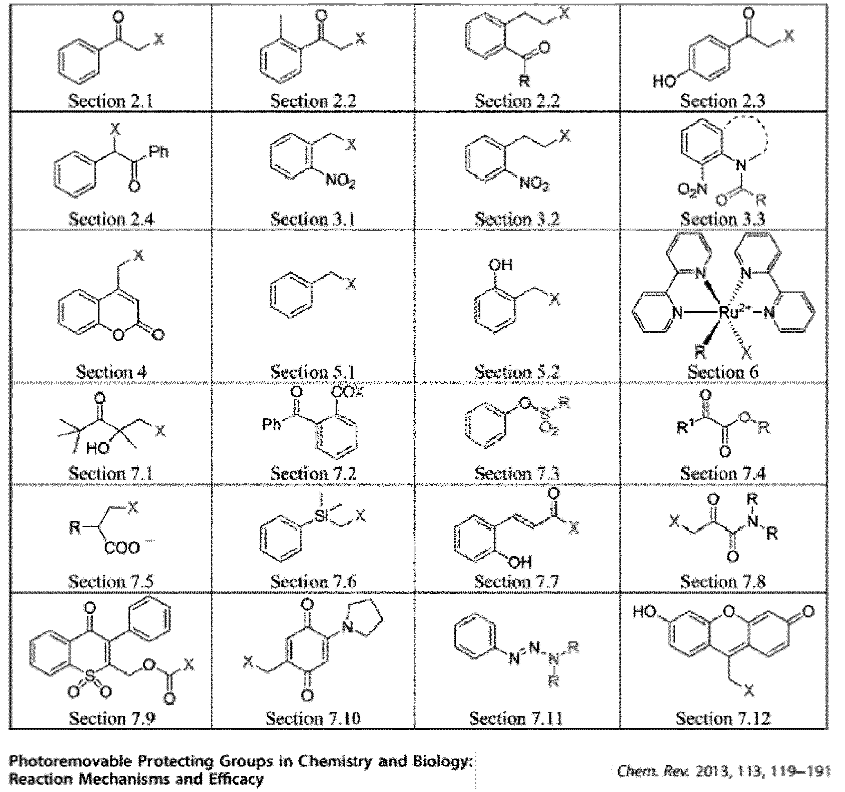

The wavelength at which the photodegradable linkers are degraded substantially equals to the absorption wavelength of the molecules.

For example, in a case of the methoxynitrobenzyl group used in the photodegradable linkers, the absorption at 346 nm is regarded as 1. The absorption at 364 nm corresponds to 0.89, the absorption at 406 nm corresponds to 0.15, and the absorption at 487 nm corresponds to 0.007. In other words, it has nature that the photodegradable linkers are degraded efficiently by using the light source having 365 nm and is almost not degraded by using the light source having 488 nm.

Thus, the wavelength of light irradiated to the photodegradable linkers may correspond to the wavelength of each photodegradable linker. For example, the wavelength is about 330 to 450 nm. In addition, the light is preferably irradiated by 30mW/cm^2, 100 sec. 3J/cm^2 that does not cause damage. In particular, the wavelength of 300 nm or less may damage the cells and is thus preferably not used.

In relation to cytotoxicity by UV, it is said that DNAs are damaged by 500 J/cm^2 and a cell growth is inhibited depending on the types of the cells (Callegari, A. J. & Kelly, T. J. Shedding light on the DNA damage checkpoint. Cell Cycle 6, 660-6 (2007)). Also, it is reported that no cytotoxicity is generated by 42J/cm^2 (Masato T, et al, Optical cell separation from three-dimensional environment in photodegradable hydrogels for pure culture techniques, Scientific Reports 4, Article number. 4793(2014)).

The first molecules 104 according to the present technology have sites bondable to the cells. As the sites bondable to the cell, an oleyl group, an antibody, an aptamer, a molecular recognition polymer, or the like can be used.

The oleyl group is hydrophobic and adheres to a surface of a floating cell. A spacer such as PEG, for example, is added to the oleyl group, an NHS group (N-hydroxysuccinimid group) is included in the end thereof, and each first molecule may be thus formed.

The antibody bonds to a cell surface molecular antigen present in each target cell to be cultured. Examples include an antibody against a variety of cancer specific antigen, an antibody against major histocompatibility antigen, an antibody against carbohydrate, and the like.

The aptamer is a nucleic acid molecule or a peptide that is specifically bonded to the molecule of each target cell to be cultured. Examples include a DNA aptamer, an RNA aptamer, a peptide aptamer, a modified aptamer having improved specificity by transfecting a modifier to a nucleic acid skeleton or a base, and the like.

The molecular recognition polymer traps a target cell surface molecule with high selectivity even in the presence of a compound having a physicochemical property similar to the cell surface molecule of each target cell to be cultured. The molecular recognition polymer is also called as a molecular imprint polymer and has a selectively synthesized compound recognition region.

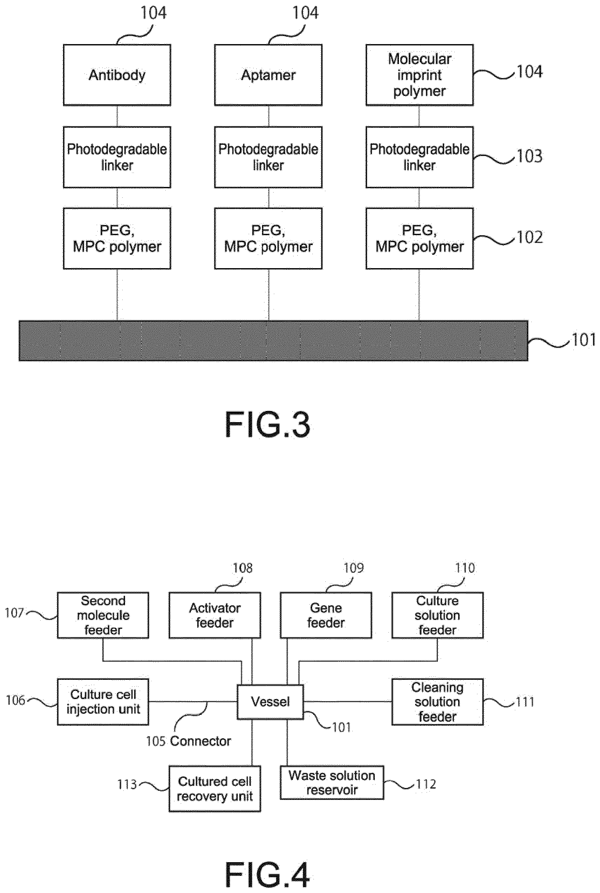

Fig. 3 shows an example of the first molecules 104 immobilized within the vessel 101. The first molecules 104 are immobilized on a bottom surface of the vessel 101 via the polymers 102 and the stimulus degradable linkers 103. In Fig. 3, the first molecules 104 are directly bonded to the stimulus degradable linkers 103 but may be bonded thereto via a polymer.

By employing the above-described structure, in a case where the first molecules 104 approach and come in contact with the cells injected into the vessel 101, the first molecules 104 can trap the cells.

The first molecules 104 are preferably immobilized to the vessel 101 in an array having a spot having a size of bonding to one target cell to be cultured. As one cell is trapped for one spot, a single cell including molecules (antibodies, carbohydrates, etc.) that are bondable to the first molecules 104 can be sorted for every spot.

A method of spotting in an array includes a micro contact printing method, a spotting method, a method of arranging over the entire surface by utilizing characteristics of the photodegradable linkers and then degrading unnecessary portions, and the like. In the portion where the cells are not to be trapped, it is preferable that the PEG and the MPC polymer are treated so as to coat or bond the inside of the vessel 101 and to inhibit non-specific adsorption of the cells. Then, the photodegradable polymer is irradiated with light to separate the unnecessary cell. Thereafter, the PEG and the MPC polymer preferably remain such that the cells are not non-specifically adsorbed on the separated spot.

Note that the first molecules to be spotted may be of one kind such that only cells of one kind are specifically trapped.

Alternatively, the first molecules each having different specificity for a spot are immobilized, and the cells different for the spot may be trapped.

Also, if the vessel 101 is partitioned and the first molecules each having different specificity for each partition are spotted, the cells that are different for each partition can be trapped in the same vessel.

Alternatively, the first molecules that trap any kinds of cells are immobilized and the cells may be sorted by the labelled second molecules described later. By utilizing a plurality of kinds of the labelled second molecules, a multicolor analysis is possible.

The vessel 101 has a variable volume. Preferably, a variable part of the vessel 101 is formed of a flexible material and can be expanded and contracted up and down and/or left and right. The volume may be varied by adjusting an amount of solution, an amount of air, or the like entered through the connector 105 of the vessel 101.

After a sample containing the target cells to be cultured is injected into the vessel 101, in order to allow the first molecules 104 to efficiently trap the target cells to be cultured, the vessel 101 preferably has a small volume. As the volume is small,
a probability of contact between the target cells to be cultured and the first molecules 104 is high.

In addition, after the target cells to be cultured trapped by the first molecules 104 are cultured and a cell density is increased, the vessel 101 preferably has an increased volume. If the volume is increased, a cell culture space is increased and the culture solution can be further added.

Note that the cell culture needs feed of oxygen and an emission of carbon dioxide. In order to provide the environment suitable to the cell culture (optimal CO₂ concentration, optimal temperature, or the like), the vessel 101 preferably has gas permeability. In particular, the surface on which the cells are grown preferably is formed of a porous film, or an oxygen permeability film, for example.

The vessel 101 may be connected to a vessel including the sample containing the target cells to be cultured and the culture solution via the connector 105.

For example, as shown in Fig. 4, the vessel 101 can be connected to any one of or a plurality of a culture cell injection unit 106, a second molecule feeder 107, an activator feeder 108, a gene feeder 109, a culture solution feeder 110, a cleaning solution feeder 111 and a waste solution reservoir 112, and a cultured cell recovery unit.

The whole may be set up under the conditions suitable for the cell culture and only the vessel 101 may be set up under the conditions suitable for the cell culture.

The culture cell injection unit 106 holds sample solution containing the target cells to be cultured and injects the sample solution into the vessel 101. The kinds of the target cells to be cultured are not especially limited and may be any of human derived cells, animal derived cells, vegetable derived cells, microorganisms derived cells, cancer cells, normal cells, stem cells, epithelial cells and the like.

The second molecule feeder 107 holds the second molecules inside and injects the second molecules into the vessel 101. In a case where a plurality kinds of second molecules are necessary, the number of the second molecule feeder 107 can be increased.

The second molecules are bondable to the target cells to be cultured and are labelled by a fluorescent material or the like. The first molecules and the second molecules form a sandwich structure of the target cells to be cultured, which can be recognized by the label.

The molecules bondable to the target cells to be cultured can be selected as sites bondable to the cell from the group consisting of an oleyl group, an antibody, an aptamer, and a molecular recognition polymer, for example, similar to the first molecules. The second molecules can be specifically bonded only to the desired cell(s) among the cells trapped by the first molecules.

In relation to the labeling of the second molecules, one kind of a fluorescent material is used to recognize one kind of the target cells to be cultured, for example. Alternatively, a plurality of kinds of fluorescent materials are used to recognize a plurality of kinds of the target cells to be cultured. In other words, the so-called multi-color analysis method may be used.

With respect to the cells which cannot be recognized by the second molecules, stimuli are added to the stimulus degradable linkers in the spot where the cells are trapped. The linkers are cut by the stimuli to separate the cells. The separated cells can be cleaned away with cleaning solution from the vessel 101 as unnecessary materials.

The cleaning solution feeder 111 holds the cleaning solution. The cleaning solution is fed when the unnecessary materials or the like is cleaned in the vessel 101.

The cleaning solution may be one commonly used in the cell culture or the like and is not especially limited. Examples include saline solution, Tris buffer solution, HEPES buffer solution, purified water, and the like.

The activator feeder 108 holds activator solution that activates the cells. The activator can be selected depending on the target cells to be cultured and is not especially limited. Examples include a variety of cytokines, antibodies, and the like. Before the target cells to be cultured are cultured, the cells can be activated.

The gene feeder 109 holds the genes to be transfected to the target cells to be cultured. The genes may be any of endogenous genes and foreign genes, and may be incorporated into a phage vector, a plasmid vector, a viral vector, or the like that is suitable to gene transfection. For example, the viral vector into which the target gene is incorporated is injected into the vessel 101 and is infected with the target cells to be cultured, and the gene transfection can be performed.

The culture solution feeder 110 holds culture solution suitable to the target cells to be cultured and feeds it to the vessel 101. The culture solution can be selected from one suitable to the target cells to be cultured. Examples include an Egle medium, a D-MEM medium, an E-MEM medium, an RPMI-1640 medium, Dulbecco PBS medium, and the like.

Note that if the culture solution is colored with Phenol Red or the like, an optimal range of the pH of the culture solution (for example, pH 6.8 to 7.2) can be controlled during the target cells to be cultured are cultured in the vessel 101.

The waste solution reservoir 112 receives waste solution containing the unnecessary materials, the culture solution, and the like for the moment. The waste solution is sterilized and discarded as necessary.

The cultured cell recovery unit 113 recovers and holds the cells cultured in the vessel 101. The method of recovering is not especially limited. It is possible to suck, extrude, arrange the cultured cell recovery unit 113 downward the vessel 101, or the like.

The connector 105 connects the vessel 101 and any one of the units 106 to 113 or a plurality of units, and the solution flows. As the connector 105, a tube is used, for example. As the preferable method of feeding the solution, the Peristaltic pump that does not come in contact with the solution is used.

### <1-2. Stimulus imparting device>

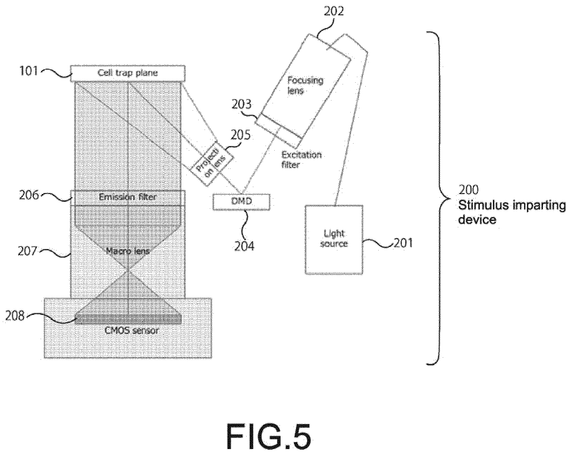

Fig. 5 shows an example of the stimulus imparting device according to the present technology. The example of the stimulus imparting device is used in a case where the stimulus degradable linkers are the photodegradable linkers.

The stimulus imparting device 200 includes a light source 201, a focusing lens 202, an excitation filter 203, a digital mirror device 204, a projection lens 205, an emission filter 206, a macro lens 207, and an image sensor 208.

By including the above-described structure, it is possible to analyze, select, and separate the unnecessary cells.

The light source 201 emits light having a wavelength corresponding to the photodegradable linker. The focusing lens 202 focuses the light, and the excitation filter 203 extracts and transmits only the light having a specific wavelength.

The digital mirror device 204 includes movable micromirrors. By tilting each micromirror, each spot where the first molecules are immobilized can be selectively irradiated with light.

The projection lens 205 irradiates the light reflected by the digital mirror device 204 toward the surface of the vessel 101 having the spot where the first molecules are immobilized.

By the selective light irradiation by the stimulus imparting device, the stimulus degradable linkers of the spot where the cells to be separate are trapped can be selectively degraded.

The light irradiated toward the surface of the vessel 101 is transmitted through the emission filter 206, the macro lens 207, and the like. The image sensor 208 receives the light and performs imaging. Imaged data can be used to verify whether or not the cells to be separated from the cells bonded to the first molecules are separated, whether or not the target cells to be cultured maintains bonding to the first molecules, or the like, for example.

The stimulus imparting device may include a stimulus controller that allows the stimulus degradable linkers to be stimulated for the first spot.

In a case where the stimulus imparting device is a light irradiation device, respective cells arranged in the vessel 101 at several tens µm orders may be irradiated, for example. For example, a Digital Micromirror Device (DMD), a liquid crystal panel, an MEMS shutter or the like can be used. An excitation filter 203 is arranged between the light source 201 and the DMD 204, and the emission filter 206 is arranged between an excitation/fluorescence object and the image sensor 208. The multi-color analysis is available by including a mechanism that the filters are rotated so as to provide an optimal filter structure depending on the object. The DMD including a full HD numbers of micromirrors is commercially available. The stimulus controller of the light irradiation device using the DMD can control (turn ON/OFF of irradiation) 1920×1080 sites at the same time. Thus, about 2×10⁶ cells can be individually controlled at the same time. For example, in a case where 1920×1080 cells (Φ30 um or less) are aligned in a 30 µm pitch, it requires a surface area of about 58×33 mm. If 10⁷ cells are to be treated, 10 surfaces are prepared. If five surfaces are arranged in two rows, it equals to 116×165 mm, which is one size smaller than B6. It is a possible size to analyze 2×10⁷ cells.

Furthermore, the stimulus imparting device according to the present technology can be used to detect the labelled second molecules. For example, the light source 201 irradiates the light having the wavelength corresponding to the fluorescent material used for labelling the second molecules to the vessel 101, imaging is performed, and it can analyze which spot traps the cells. In addition, if a plurality of fluorescent materials are used, the multi-color analysis is available to analyze which cells are trapped on which spots.

As described above, the cell culture system according to the present technology can analyze, sort, process, culture, and control quality of the cells in the same space.

### <2. Embodiments>

Hereinafter, illustrative embodiments will be described.

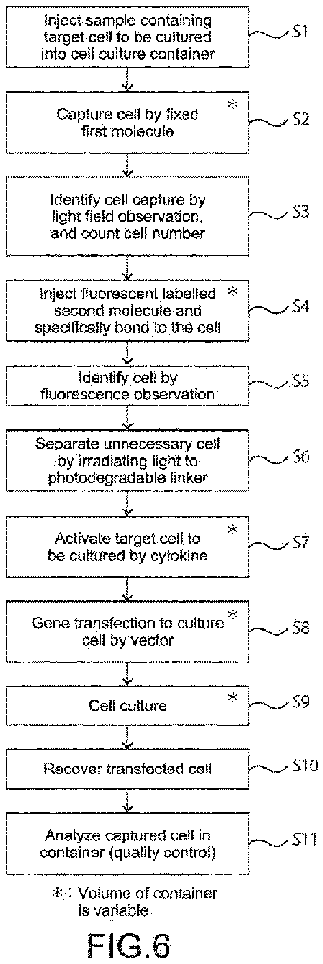

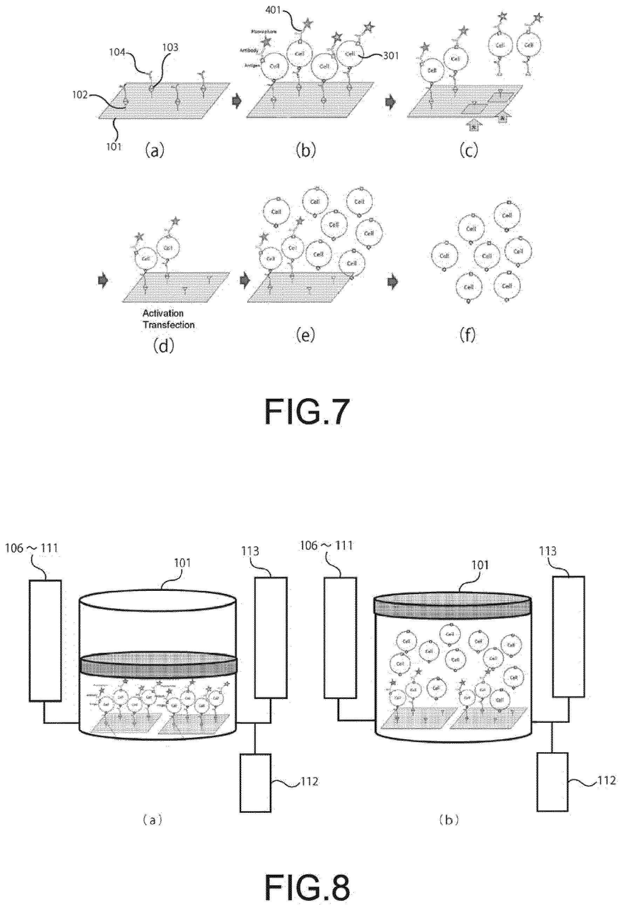

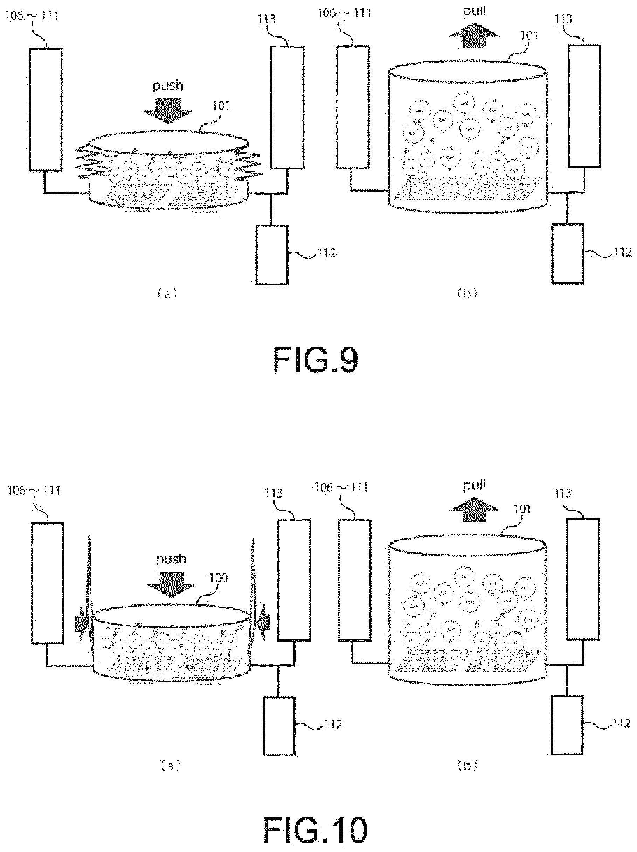

Hereinafter, with reference to a block diagram showing respective steps according to the embodiment of Fig. 6, schematic diagrams of the cell culture container according to the embodiment of Fig. 7, schematic diagrams showing a volume variation of the cell culture container of Fig. 8, and schematic diagrams showing movements of the cell culture container of Fig. 9 and Fig. 10, the description will be made.

To a two-dimensional surface, i.e., the bottom surface of the vessel 101, antibodies, i.e., the first molecules 104, are bonded via the photodegradable linkers, i.e., the polymers 102 and the stimulus degradable linkers 103 (Fig. 7(a)).

Here, a sample containing target cells to be cultured is injected from the culture cell injection unit 106 (S1). The first molecules 104 specifically trap cells 301 (S2). This is an initial cell selection. The cells not bonded to the first molecules 104 and other unnecessary materials are flowed downstream by the cleaning solution from the cleaning solution feeder 111 and are reserved in the waste solution reservoir 112.

At this stage, a cell trap may be identified by light field observation by a microscope or the like, for example, and a trapped cell number may be determined (S3).

Next, the antibodies, i.e., the second molecules 401 labelled by the fluorescent material, are injected from the second molecule feeder 107 and are specifically bonded to the cells 301 trapped by the first molecules 104 (S4). This forms the sandwich structure of the first molecules 104, the cells 301 and the second molecules 401 (Fig. 7(b)). Non-bonded second molecules can be cleaned and removed. Next, fluorometry is performed on fluorescent labeling of the second molecules 401 to identify the trapped cells 301 (S5).

At this time, in a case where a plurality kinds of second molecules are used to sort the cells, fluorescence is detected or a fluorescence intensity is measured by bonding a plurality of antibodies labelled with different fluorescences to the cells 301 trapped by the first molecules 104. The so-called multi-color analysis is possible. In the fluorometry, by changing an excitation filter or a fluorescence filter, the detection or the measurement is possible corresponding to a variety of fluorescence colors.

Note that in the step of bonding the first molecules 104 and the cells and the step of bonding the second molecules 401 and the cells, the vessel 101 has the decreased volume as shown in Fig. 8(a), the chances to come in contact each other is increased, resulting in efficient bonding. A variable space of the vessel 101 may be formed by arranging a port near the bottom surface, descending an upper part of the vessel in the steps other than the culture, and by ascending the vessel to increase the volume. Other methods include decreasing the volume by clamping the vessel, descending a hard upper surface to suppress with a side pressure, and the like, for example.

Here, Fig. 9 and Fig. 10 show illustrative structures to vary the volume of the vessel 101.

Fig. 9 shows an example having a bellows structure on the side of the vessel 101. Fig. 9(a) shows a situation that a pressure is applied from the upper surface of vessel 101, the bellows is contracted, and the volume is decreased. Fig. 9(b) shows a situation that a force of pulling the upper surface of the vessel 101 is applied, the bellows is elongated, and the volume is increased.

Fig. 10 shows an example that the upper surface and the side surface of the vessel 101 are pushed to vary the volume. Fig. 10(a) shows a situation that the upper surface and the side surface of the vessel 101 are pushed to decrease the volume. Fig. 10(b) shows a situation that a force of pulling the upper surface of the vessel 101 is applied and the volume is increased.

Note that the steps other than the cell culture are preferably performed in a space as narrow as possible because reaction efficiency is good and there is no waste of the reagents. For example, when the cells are trapped, the cells are activated, and the genes are transfected, a narrow space is provided by decreasing the height. On the other hand, when the cells are cultured, a space for increased cells is necessary and a wide space is desirable. At the time of the cell culture, there is an expansion culture that the cells are moved from the small space to the big space. The movement may cause a damage or a loss. Accordingly, according to the present technology, it is desirable that the culture can be performed in the same space.

After the cells 301 are sandwiched between the first molecules 104 and the second molecules 401, fluorescence identification is performed. In a case where the cells are determined to not be target cells to be cultured (unnecessary cells), light is irradiated to the photodegradable linkers of the spots where the cells are trapped using the stimulus imparting device 200, the linkers are degraded, and the cells are separated (S6, Fig. 7(c)).

The selection of the photodegradable linkers used here is preferably not overlapped with an excitation wavelength used for the fluorometry. Since the excitation wavelength of 405 nm to 638 nm is often used, other wavelengths are preferable. In addition, a short wavelength may cause cytotoxicity or damages to some cells. Depending on the type of the target cells, it is necessary to use an optimal photodegradable linker.

In addition, the light irradiation device using the above-described DMD can be used not only upon the separation of the cells, but also upon the detection by fluorescence. Since only the target cells can be irradiated with light, an advantage is a decrease in overall background noise. As a result, an S/N is increased. Furthermore, if all adjacent cells are excited and a cross talk is concerned, it is possible to avoid the cross talk by divided irradiation.

After the light is irradiated to degrade the photodegradable linkers and separate the unnecessary cells, the unnecessary cells are cleaned and removed from the vessel 101.

The target cells to be cultured remain on the bottom surface of the vessel 101. Appropriate cell processing is performed on the cells (Fig. 7(d)). Examples of the cell processing include: feeding the above-described cytokines, the antibodies that impart stimuli, and the like from the activator feeder 108 to thereby activating the cells (S7), injecting viral vectors or the like from the gene feeder 109 to thereby performing the gene transfection (S8), and the like.

Next, the target cells to be cultured are cultured (S9, Fig. 7(e)).

The culture solution is fed to the vessel 101 from the culture solution feeder 110. For example, if human or animal cells are cultured, the preferable conditions are the CO₂ concentration of 5%, the humidity of 90 to 95%, and the temperature of 37°C.

At the time of culture, the vessel 101 preferably has an increased volume as shown in Fig. 8(b).

After the cell culture, the cells are recovered to other vessel, e.g., the cultured cell recovery unit 113 connected to the vessel 101 via the connector 105 (S10, Fig. 7(f)). At this time, the cells remain trapped at the bottom surface of the vessel 101. The cells are again bonded to the fluorescent labelled antibodies (labelled second molecules), a fluorescence analysis is performed, and a quality control can be thus performed (S11).

Thus, according to the present technology, cell sorting, processing (activation, gene transfer, etc.), culture, and quality control can be performed in the same space.

### <3. Cell culture kit>

A cell culture kit according to the present technology includes the vessel 101, and any of sample solution containing the target cells to be cultured, reagent solution containing the second molecules, activator solution containing an activator, gene transfection solution containing genes transfected, culture solution, and the cleaning solution, a plurality of the solutions, or all solutions.

If these solutions are sealed in a pack, for example, the entire pack is disposable and replaceable as necessary, and is easily transported and stored, and a contamination or the like can be prevented.

### Reference Signs List

- 100: cell culture container
- 101: vessel
- 102: polymer
- 103: stimulus degradable linker
- 104: first molecule
- 105: connector
- 106: culture cell injection unit
- 107: second molecule feeder
- 108: activator feeder
- 109: gene feeder
- 110: culture solution feeder
- 111: cleaning solution feeder
- 112: waste solution reservoir
- 113: cultured cell recovery unit
- 200: stimulus imparting device
- 201: light source
- 202: focusing lens
- 203: excitation filter
- 204: digital mirror device

- 205: projection lens
- 206: emission filter
- 207: macro lens
- 208: image sensor
- 301: cell
- 401: second molecule
- 1000: cell culture system

## Claims

1. A cell culture container (100), comprising:
first molecules (104) each bondable to target cells to be cultured, the first molecules (104) being immobilized to the container (100) via a stimulus degradable linker (103), the container (100) having a volume being variable.

2. The cell culture container (100) according to claim 1, wherein
the stimulus degradable linker (103) is a photodegradable linker.

3. The cell culture container (100) according to claim 1, wherein
the first molecules (104) are immobilized to the container (100) in an array having a spot with a size for bonding to one target cell to be cultured.

4. The cell culture container (100) according to claim 3, wherein
each of the first molecules (104) bonds to a different kind of the target cells for the spot.

5. The cell culture container (100) according to claim 1, wherein
the cell culture container (100) has a gas permeability.

6. The cell culture container (100) according to claim 1, wherein
the first molecules (104) are selected from the group consisting of an oleyl group, an antibody, an aptamer, and a molecular recognition polymer.

7. The cell culture container (100) according to claim 1, further comprising:
a connector (105) that is connected to one selected from the group consisting of
a culture cell injection unit (106) that injects solution containing the target cells,
a second molecule feeder (107) that feeds labelled second molecules (401) bondable to the target cells,
an activator feeder (108) that feeds an activator of activating the target cells,
a gene feeder (109) that feeds genes to be transfected to the target cells,
a culture solution feeder (110) that feeds culture solution of the target cells,
a cleaning solution feeder (111) that feeds cleaning solution of cleaning away an unnecessary material,
a waste solution reservoir (112) that reserves the unnecessary material and the cleaning solution, and
a cultured cell recovery unit (113) that recovers cultured cells of the target cells.

8. A cell culture system (1000), comprising:
a cell culture container (100), including
first molecules (104) each bondable to target cells to be cultured, the first molecules (104) being immobilized to the container (100) via a stimulus degradable linker (103), the container (100) having a volume being variable; and
a stimulus imparting device (200) that imparts a stimulus to the stimulus degradable linker (103).

9. The cell culture system (1000) according to claim 8, wherein
the stimulus imparting device (200) includes a light source (201), an excitation filter (203), an emission filter (206), and an image sensor (208).

10. The cell culture system (1000) according to claim 8, wherein
the stimulus imparting device (200) includes a stimulus controller that controls stimulation of the stimulus degradable linker (103) for a spot of the first molecule (104).

11. A cell culture kit, comprising:
a cell culture container (100), including
first molecules (104) each bondable to target cells to be cultured, the first molecules (104) being immobilized to the container (100) via a stimulus degradable linker (103), the container (100) having a volume being variable; and
solution selected from the group consisting of
sample solution containing the target cells, reagent solution containing labelled second molecules (401) bondable to the target cells,
activator solution containing an activator that activates the target cells,
gene transfection solution containing genes transfected to the target cells,
culture solution of the target cells, and cleaning solution that cleans away unnecessary material.

12. A cell culture method, comprising the steps of:
injecting (S1) a sample containing target cells to be cultured into a cell culture container (100), including
first molecules (104) each bondable to target cells, the first molecules (104) being immobilized to the container (100) via a stimulus degradable linker (103), the container (100) having a volume being variable;
applying labelled second molecules (401), being bondable to the target cells, to the target cells;
sorting the target cells by the label and removing cells other than the target cells; and
culturing the target cells.

13. The cell culture method according to claim 12, further comprising the steps of:
activating (S7) the target cells; and/or
transfecting (S8) genes to the target cells.

14. The cell culture method according to claim 12, wherein
at least one of the steps includes changing the volume of the container (100).

## Patentansprüche

1. Zellkulturbehälter (100), umfassend:
erste Moleküle (104), die jeweils an zu kultivierende Zielzellen bindungsfähig sind, wobei die ersten Moleküle (104) an dem Behälter (100) mittels eines stimulusabbaubaren Linkers (103) immobilisiert sind, wobei der Behälter (100) ein Volumen aufweist, das variabel ist.

2. Zellkulturbehälter (100) nach Anspruch 1, wobei der stimulusabbaubare Linker (103) ein photoabbaubarer Linker ist.

3. Zellkulturbehälter (100) nach Anspruch 1, wobei die ersten Moleküle (104) in einer Gruppierung, die einen Spot mit einer Größe zum Binden an eine zu kultivierende Zielzelle aufweist, an dem Behälter (100) immobilisiert sind.

4. Zellkulturbehälter (100) nach Anspruch 3, wobei jedes der ersten Moleküle (104) an eine andere Art der Zielzellen für den Spot bindet.

5. Zellkulturbehälter (100) nach Anspruch 1, wobei der Zellkulturbehälter (100) eine Gaspermeabilität aufweist.

6. Zellkulturbehälter (100) nach Anspruch 1, wobei die ersten Moleküle (104) ausgewählt sind aus der Gruppe bestehend aus einer Oleylgruppe, einem Antikörper, einem Aptamer und einem Molekülerkennungspolymer.

7. Zellkulturbehälter (100) nach Anspruch 1, des Weiteren umfassend:
einen Konnektor (105), der mit einem ausgewählt aus der Gruppe bestehend aus folgenden verbunden ist:
einer Kulturzellinjektionseinheit (106), die Lösung injiziert, welche die Zielzellen enthält,
einer zweiten Moleküleinspeisung (107), die markierte zweite Moleküle (401) einspeist, die an die Zielzellen bindungsfähig sind,
einer Aktivatoreinspeisung (108), die einen Aktivator einspeist, um die Zielzellen zu aktivieren,
einer Geneinspeisung (109), die Gene einspeist, die in die Zielzellen transfiziert werden sollen
einer Kulturlösungseinspeisung (110), die Kulturlösung für die Zielzellen einspeist,
einer Reinigungslösungseinspeisung (111), die Reinigungslösung einspeist, um nicht notwendiges Material durch Reinigung zu entfernen,
einem Abfalllösungsreservoir (112), welches das nicht notwendige Material und die Reinigungslösung verwahrt, und
einer Gewinnungseinheit für kultivierte Zellen (113), die kultivierte Zellen der Zielzellen gewinnt.

8. Zellkultursystem (1000), umfassend:
einen Zellkulturbehälter (100), der einschließt:
erste Moleküle (104), die jeweils an zu kultivierende Zielzellen bindungsfähig sind, wobei die ersten Moleküle (104) an dem Behälter (100) mittels eines stimulusabbaubaren Linkers (105) immobilisiert sind, wobei der Behälter (100) ein Volumen aufweist, das variabel ist; und
eine stimulusverleihende Vorrichtung (200), die dem stimulusabbaubaren Linker (103) einen Stimulus verleiht.

9. Zellkultursystem (1000) nach Anspruch 8, wobei die stimulusverleihende Vorrichtung (200) eine Lichtquelle (201), ein Anregungsfilter (203), ein Emissionsfilter (206) und einen Bildsensor (208) einschließt.

10. Zellkultursystem (1000) nach Anspruch 8, wobei die stimulusverleihende Vorrichtung (200) eine Stimulussteuerung einschließt, welche die Stimulation des stimulusabbaubaren Linkers (105) für einen Spot des ersten Moleküls (104) steuert.

11. Zellkulturkit, umfassend:
einen Zellkulturbehälter (100), der einschließt:
erste Moleküle (100), die jeweils an zu kultivierende Zielzellen bindungsfähig sind, wobei die ersten Moleküle (104) an dem Behälter (100) mittels eines stimulusabbaubaren Linkers (105) immobilisiert sind, wobei der Behälter (105) ein Volumen aufweist, das variabel ist; und
Lösung ausgewählt aus der Gruppe bestehend aus:
Probenlösung, welche die Zielzellen enthält, Reagenzlösung, welche markierte zweite Moleküle (401) enthält, die an die Zielzellen bindungsfähig sind,
Aktivatorlösung, die einen Aktivator enthält, der die Zielzellen aktiviert,
Gentransfektionslösung, die Gene enthält, die in die Zielzellen transfiziert werden, Kulturlösung der Zielzellen und Reinigungslösung, die nicht notwendiges Material durch Reinigung entfernt.

12. Zellkulturverfahren, umfassend die Schritte:
Injizieren (S1) einer Probe, die zu kultivierende Zielzellen enthält, in einen Zellkulturbehälter (100), der einschließt:
erste Moleküle (104), die jeweils an Zielzellen bindungsfähig sind, wobei die ersten Moleküle (104) an dem Behälter (100) mittels eines stimulusabbaubaren Linkers (105) immobilisiert sind, wobei der Behälter (100) ein Volumen aufweist, das variabel ist;
Applizieren von markierten zweiten Molekülen (401), die an die Zielzellen bindungsfähig sind, auf die Zielzellen;
Sortieren der Zielzellen gemäß der Markierung, und Entfernen von Zellen, welche von den Zielzellen verschieden sind; und
Kultivieren der Zielzellen.

13. Zellkulturverfahren nach Anspruch 12, des Weiteren umfassend die Schritte:
Aktivieren (S7) der Zielzellen; und/oder Transfizieren (S8) von Genen in die Zielzellen.

14. Zellkulturverfahren nach Anspruch 12, wobei mindestens einer der Schritte das Ändern des Volumens des Behälters (100) einschließt.

## Revendications

1. Récipient de culture cellulaire (100), comprenant :
des premières molécules (104), chacune pouvant être liée à des cellules cibles à cultiver, les premières molécules (104) étant immobilisées contre le récipient (100) par le biais d'un lieur dégradable par stimulus (103), le récipient (100) ayant un volume variable.

2. Récipient de culture cellulaire (100) selon la revendication 1, dans lequel
le lieur dégradable par stimulus (103) est un lieur photodégradable.

3. Récipient de culture cellulaire (100) selon la revendication 1, dans lequel
les premières molécules (104) sont immobilisées contre le récipient (100) en un réseau ayant un point avec une taille permettant la liaison à une cellule cible à cultiver.

4. Récipient de culture cellulaire (100) selon la revendication 3, dans lequel
chacune des premières molécules (104) se lie à un type différent des cellules cibles pour le point.

5. Récipient de culture cellulaire (100) selon la revendication 1,
le récipient de culture cellulaire (100) ayant une perméabilité aux gaz.

6. Récipient de culture cellulaire (100) selon la revendication 1, dans lequel
les premières molécules (104) sont choisies dans le groupe constitué par un groupe oléyle, un anticorps, un aptamère, et un polymère de reconnaissance moléculaire.

7. Récipient de culture cellulaire (100) selon la revendication 1, comprenant en outre :
un raccord (105) qui est raccordé à un élément choisi dans le groupe constitué par
une unité d'injection de culture cellulaire (106) qui injecte une solution contenant les cellules cibles,
un distributeur de deuxièmes molécules (107) qui distribue des deuxièmes molécules marquées (401) pouvant être liées aux cellules cibles,
un distributeur d'activateur (108) qui distribue un activateur d'activation des cellules cibles,
un distributeur de gènes (109) qui distribue des gènes à transfecter dans les cellules cibles,
un distributeur de solution de culture (110) qui distribue une solution de culture des cellules cibles,
un distributeur de solution nettoyante (111) qui distribue une solution nettoyante de retrait de matière inutile,
un réservoir de solution de déchets (112) qui conserve la matière inutile et la solution nettoyante, et
une unité de récupération de cellules cultivées (113) qui récupère des cellules cultivées des cellules cibles.

8. Système de culture cellulaire (1000), comprenant :
un récipient de culture cellulaire (100), comportant
des premières molécules (104), chacune pouvant être liée à des cellules cibles à cultiver, les premières molécules (104) étant immobilisées contre le récipient (100) par le biais d'un lieur dégradable par stimulus (105), le récipient (100) ayant un volume variable ; et
un dispositif d'application de stimulus (200) qui applique un stimulus au lieur dégradable par stimulus (103) .

9. Système de culture cellulaire (1000) selon la revendication 8, dans lequel
le dispositif d'application de stimulus (200) comporte une source de lumière (201), un filtre d'excitation (203), un filtre d'émission (206), et un capteur d'images (208).

10. Système de culture cellulaire (1000) selon la revendication 8, dans lequel
le dispositif d'application de stimulus (200) comporte un contrôleur de stimulus qui contrôle la stimulation du lieur dégradable par stimulus (105) pour un point de la première molécule (104).

11. Trousse de culture cellulaire, comprenant :
un récipient de culture cellulaire (100), comportant
des premières molécules (100), chacune pouvant être liée à des cellules cibles à cultiver, les premières molécules (104) étant immobilisées contre le récipient (100) par le biais d'un lieur dégradable par stimulus (105), le récipient (105) ayant un volume variable ; et une solution choisie dans le groupe constitué par
une solution échantillonnée contenant les cellules cibles,
une solution de réactif contenant des deuxièmes molécules marquées (401) pouvant être liées aux cellules cibles,
une solution d'activateur contenant un activateur qui active les cellules cibles,
une solution de transfection de gènes contenant des gènes transfectés dans les cellules cibles,
une solution de culture des cellules cibles, et
une solution nettoyante qui retire la matière inutile.

12. Procédé de culture cellulaire, comprenant les étapes suivantes :
injection (S1) d'un échantillon contenant des cellules cibles à cultiver dans un récipient de culture cellulaire (100), comportant
des premières molécules (104), chacune pouvant être liée à des cellules cibles, les premières molécules (104) étant immobilisées contre le récipient (100) par le biais d'un lieur dégradable par stimulus (105), le récipient (100) ayant un volume variable ;
application de deuxièmes molécules marquées (401), qui peuvent être liées aux cellules cibles, aux cellules cibles ;
tri des cellules cibles par le marqueur et retrait des autres cellules que les cellules cibles ; et
culture des cellules cibles.

13. Procédé de culture cellulaire selon la revendication 12, comprenant en outre les étapes suivantes :
activation (S7) des cellules cibles ; et/ou transfection (S8) de gènes dans les cellules cibles.

14. Procédé de culture cellulaire selon la revendication 12, dans lequel
au moins une des étapes comporte la variation du volume du récipient (100).
